# EUROPEAN PATENT SPECIFICATION

(11) **EP 3 870 099 B1**
(45) Date of publication and mention of the grant of the patent: **13.12.2023**
(21) Application number: 19877335.0
(22) Date of filing: 22.10.2019
(51) Int. Cl.: A61C 8/00, A61B 17/70, A61B 17/86

(54) **ANCHOR WHICH IS SCREWABLE**
ANKER, DER SCHRAUBBAR IST
ANCRAGE POUVANT ÊTRE VISSÉ

(30) Priority: 22.10.2018 US 201862748773 P
(43) Date of publication of application: 01.09.2021
(73) Proprietor: Huwais IP Holding LLC, Jackson, MI 49201 (US)
(72) Inventor: HUWAIS, Salah, Jackson, MI 49203 (US)
(74) Representative: Higgs, Jonathan
(86) International application number: PCT/US2019/057477
(87) International publication number: WO 2020/086611

(56) References cited:
- WO-A1-2017/136801
- US-A1- 2011 070 558
- US-A1- 2015 044 638
- US-A1- 2016 166 358
- US-A1- 2017 071 702
- US-A1- 2017 348 073
- US-A1- 2017 348 073
- US-B1- 6 402 515
- US-B2- 9 737 312

## Description

[Deleted]

### BACKGROUND OF THE INVENTION

Field of the Invention. The invention relates generally to anchors intended to provide fixation in a host material, and more particularly to anchors designed to produce compaction in the host material as the anchor is screwed into position, and even more particularly to such anchors placed in living organic materials like bone.

Description of Related Art. Screw-in anchors are used in many applications. For example, in industrial and construction settings, where the host material is wood or concrete or metal or polymer, an anchor may be placed in a wall or other member to provide a fixed point of connection to attach another element. Screw-in anchors are used extensively in medical applications, where the host material is bone, to provide a fixed point of connection for metal plates, pins, rods, Kirschner wires and intramedullary devices such as the Küntscher nail and interlocking nail, among many other uses.

Dental anchors are another form of screw-in anchor where the host material is bone. A dental anchor, also known as an endosteal implant or fixture, is a surgical device used to support a crown, bridge of teeth, denture, facial prosthetic or to act as an orthodontic anchor. Typically, such anchors are designed as threaded, tapered implants that are not loaded immediately after setting in order that full stability (i.e., secondary stability) may be reached over time as the surrounding bone grows into the crevices of and around the anchor - a process known as osseointegration. Several months may be required for bone ingrown until the anchor reaches enough (secondary) stability to be put into normal service.

In many applications, anchor stability is a key consideration because the anchor must be able to support the intended loading. When the host material is not organic, living tissue, maximum anchor stability is usually achieved immediately after placement. For these situations, the anchor should be designed to maximize initial stability, also known as primary stability. In applications where the host material is an organic living material, like bone or wood for example, reaching full anchor stability may require the passage of time for healing and in-growth after placement. In these latter cases, the faster an anchor can reach an adequate level of secondary stability, the better.

Anchors that possess an adequate level of stability at the time of initial placement are highly valued. Although the prior art is composed of a great many different designs and concepts aimed at improving anchor stability - both primary (initial) and secondary (long-term) - there remains a continuing desire for improvement. Specifically, anchor stability remains a long-felt need in the art where improvements are readily embraced. US2016/166358A1, US2017/348073A1 and WO2017/136801A1 disclose related art.

### BRIEF SUMMARY OF THE INVENTION

According to a first aspect of this invention an anchor is provided of the type to be screwed into a prepared hole in a host material. The anchor comprises a body that extends longitudinally along a central axis. The body has an apical region and a coronal region. A central region of the body extends between the apical region and the coronal region. At least one thread form protrudes from the body and winds helically there along in continuous turns from the apical region to the coronal region. The thread form has a crest. The central region includes an array of flutes. Each flute extends longitudinally along the length of the central region. Each flute is composed of a plurality of distinct and isolated flute segments formed in the crest of the thread form.

According to a second aspect of this invention a bone anchor is configured to be screwed into a prepared hole in host bone. The anchor comprises a body that extends longitudinally along a central axis. The body has an apical region and a coronal region. A central region of the body extends between the apical region and the coronal region. The body has an apical taper throughout the apical region. The apical taper is between about 5°-15° relative to the central axis. The body has a coronal taper throughout the coronel region. The coronal taper is between about 5°-15° relative to the central axis. The body has a central taper throughout the central region. The central taper is between about 0°-5° relative to the central axis. At least one thread form protrudes from the body and winds helically there along in continuous turns from the apical region to the coronal region. The thread form has a truncated crest. The central region includes an array of flutes. Each flute extends longitudinally along the length of the central region. The thread form has a leading flank disposed toward the apical region and a trailing flank disposed toward the coronal region. The leading flank has a leading flank angle between about 110°-130° measured toward the apical region. The trailing flank has a trailing flank angle between about 75°-85° measured toward the coronal region. Each flute is composed of a plurality of distinct and isolated flute segments formed in the crest of the thread form.

The flute segments create a distributed array of voids around a freshly seated anchor. Each void represents a discrete healing chamber. The condensing ramps provide a synergistic effect by creating induced stress and super-activated zones in the host material directly adj acent to the healing chambers. The healing chambers draw host material that has been naturally encouraged to promote healing (in living host material) from the adjacent induced stress and super-activated zones. Reaction forces in the host material cause the surrounding host material to constrict about the anchor and fill the healing chambers. Because the healing chambers originate as voids, there is little-to-no resistance the migration of host material into the healing chambers. In bone, super-activated zones also provoke blood flow, which leads to clots forming in the voids of the flutes 60. Clots activate bone's natural healing properties, which leads to rapid growth of new bone in the healing chambers. Thus, in bone implant applications, the super-activated zones promote the natural healing properties in the human body to accelerate recovery and improve osseointegration, especially into the healing chambers formed above each flute segment.

### BRIEF DESCRIPTION OF THE SEVERAL VIEWS OF THE DRAWINGS

These and other features and advantages of the present invention will become more readily appreciated when considered in connection with the following detailed description and appended drawings, wherein:
Figure 1 is a cross-section though an osteotomy in a human mandible with an anchor according to one embodiment of this invention fully seated;
Figure 2 is a perspective view of an anchor according to one embodiment of this invention;
Figure 3 a perspective view of the anchor of Figure 2 but from a different orientation;
Figure 4 is a front elevation of the anchor of Figures 2 and 3;
Figure 5 is a coronal end view as taken generally along lines 5-5 in Figure 4;
Figure 6 is an apical end view as taken generally along lines 6-6 in Figure 4;
Figure 7 is a longitudinal cross-section taken generally along lines 7-7 in Figure 4;
Figure 8 is a transverse cross-section taken generally along lines 8-8 in Figure 4;
Figure 9 is an isometric view as perceived from the apical end clearly showing the segmented flute features;
Figure 10 is a transverse cross-section taken generally along lines 10-10 in Figure 1;
Figure 11A is an enlarged view of the area circumscribed at 11C in Figure 10 but showing the anchor and the surrounding bone materials in a condition at the time of installation;
Figure 11B is a view as in Figure 11A but showing a time progression in which healing is evidenced by the growth of blood clots into the healing chambers;
Figure 11C is an enlarged view of the area circumscribed at 11C in Figure 10 and is also a still further time progression of Figure 11B; and
Figure 12 is an enlarged view of the area circumscribed at 12 in Figure 1.

### DETAILED DESCRIPTION OF THE INVENTION

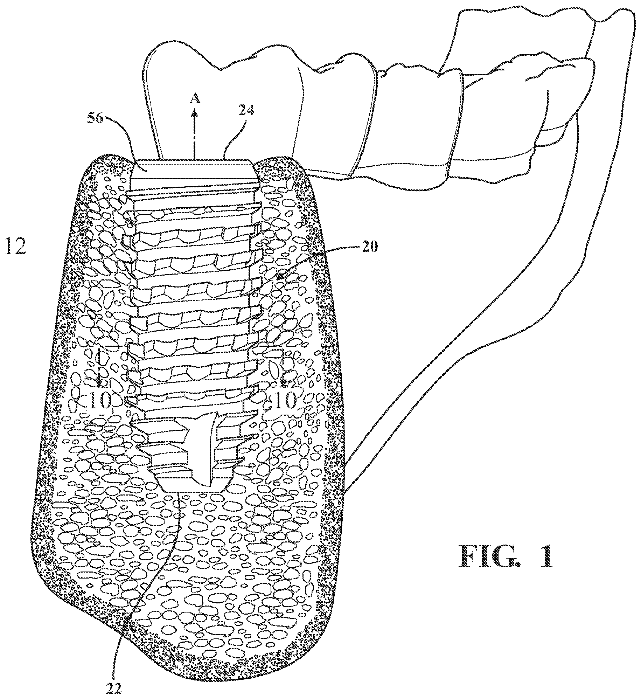

Referring to the figures, wherein like numerals indicate like or corresponding parts throughout the several views, the invention is described in the dental context, in which preparation of an osteotomy is required to receive a bone implant (Figure 1). It will be understood that this invention is not limited to dental applications but may be applied across a wide spectrum of orthopedic applications. Furthermore, the invention is not limited to bone or orthopedic applications. This invention may be used to provide an anchor in living trees and other live cellular materials, as well as metal foams and other non-living host materials used for various industrial and commercial applications, to name but a few. Nevertheless, dental applications represent a compelling example, such that the following description will make use of the dental context with bone as the host material for illustrative purposes.

In Figure 1, an edentulous jaw site is shown in cross-section having implanted therein an anchor 20 according to one embodiment of the present invention. The anchor 20 is of the type that can be screwed into a prepared hole in a host material. When the host material is bone, the prepared hole is referred to as an osteotomy.

The prepared hole or osteotomy can be formed using any suitable technique. One such technique comprises the use of progressively wider rotary osteotomes specially configured to achieve osseodensification. The procedure of forming an osteotomy using progressively wider rotary osteotomes is described, generally, in US 9,326,778 to Huwais, issued May 3, 2016. Reference may also be had to US patent publication number US20190029695A1 to Huwais, published January 31, 2019.

Once the osteotomy has been prepared, again by any suitable technique, the anchor 20 is screwed into place, as illustrated in Figure 1, by turning in the right-hand direction. An abutment (not shown) is eventually threaded into an internal connect and is thereby secured in position to receive a subsequent restoration or crown (also not shown). The anchor 20 is perhaps ideally suited for placement in bone, yet non-bone applications are contemplated to reiterate the previous assertion. To say again, although the illustrated embodiments depict the anchor 20 in the form of an implant or receptor for a dental restoration, it must be appreciated that the anchor 20 may be reconfigured as a bone screw or other bone fixation element as may be used for example in spinal, hip, shoulder, wrist and other orthopedic applications.

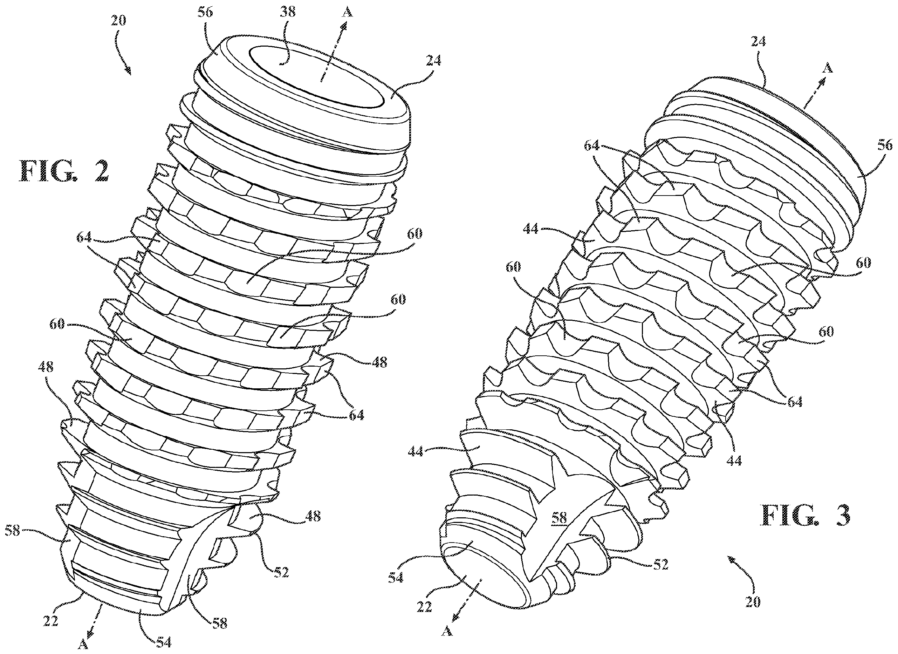

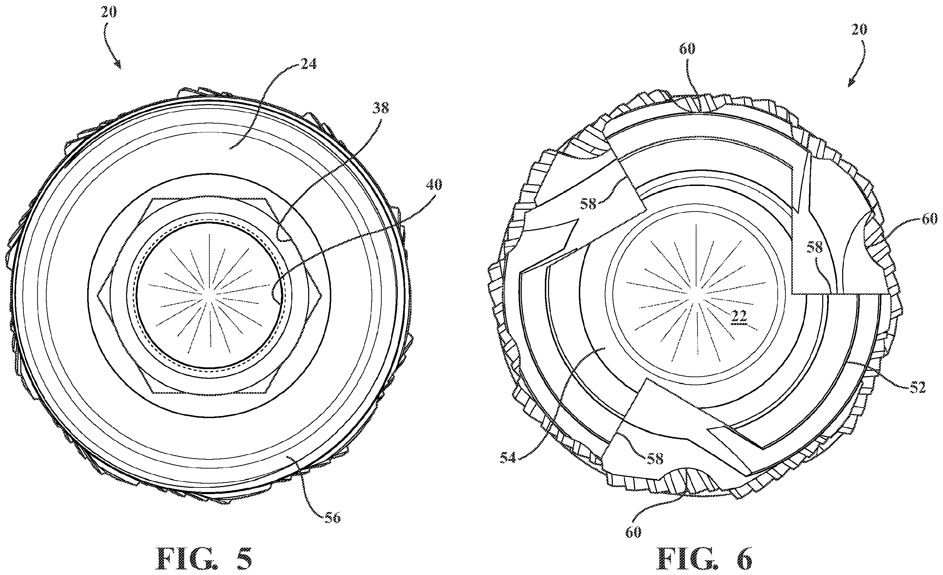

Turning now to Figures 2-4, the anchor 20 is shown in one embodiment comprising a truncated body formed with a more or less tapered outer profile. The body of the anchor 20 extends longitudinally along a central or longitudinal axis A. The body has an apical end 22 and a coronal end 24. The terms "apical" and "coronal" are selected primarily for their dental association. "Apical" means a direction toward the root tip(s) of a tooth; "coronal" means a direction toward the crown of a tooth. Applicant's use of these terms and perhaps others in this document must not be construed narrowly as to limit the application of the anchor 20 to the dental fields of use, or even to the medical fields of use. A view of the anchor 20 from the perspective of the coronal end 24 is provided in Figure 5. A view of the anchor 20 from the perspective of the apical end 22 is provided in Figure 6. The coronal end 24 is preferably flat, or substantially flat, and serves as a platform for a dental restoration or other subsequent attachment to the anchor 20. The apical end 22 forms the leading end of the anchor 20 and in use is inserted first into the prepared osteotomy. The apical end 22 may be configured with a blunt tip as shown in Figure 3 or could in the alternative be domed or pointed or indented (concave) or designed with some other suitable shape to help prevent over-insertion or otherwise contribute to safer implant placement.

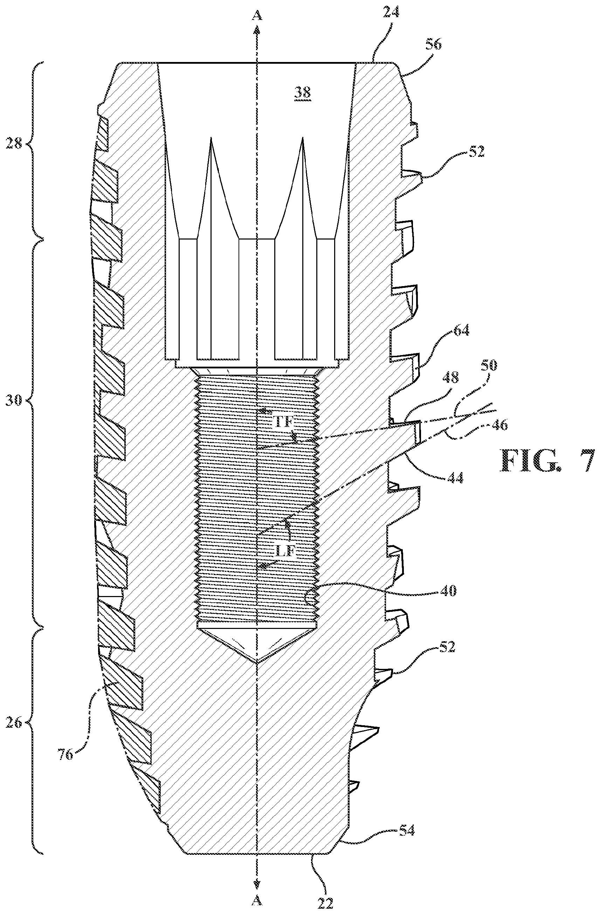

As identified in Figure 7, the anchor 20 has an apical region 26 adjacent the apical end 22, and a coronal region 28 adjacent the coronal end 24. Thus, the apical region 26 represents a portion of the body that extends from the apical end 22. And likewise, the coronal region 28 represents a portion of the body that extends from the coronal end 24. A central region 30 of the body extends between the apical region 26 and the coronal region 28. The central region 30 is contiguous, on either end, to the apical 26 and coronal 28 regions such that these three regions 26-30 fully occupy the entire longitudinal length of the body, from end 22 to end 24, in the illustrated examples.

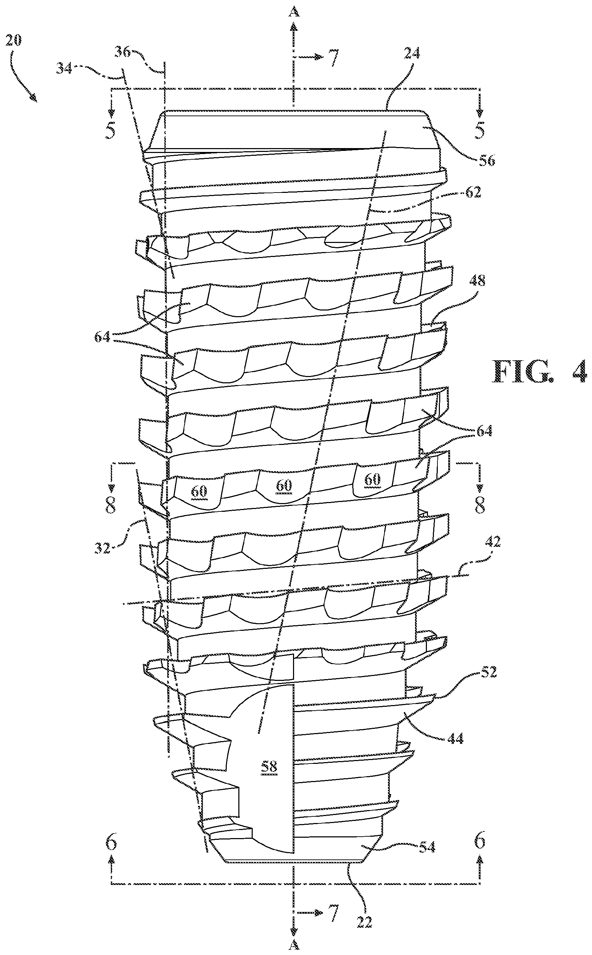

In Figure 4 the outer surface of the body can be easily discerned as having a tapered profile. In particular, the various regions 26-30 can be distinguished from one another by, among other things, the characteristics of the body taper. It therefore makes sense to describe the body as having an apical taper 32 throughout the apical region 26, a coronal taper 34 throughout the coronel region 28, and a central taper 36 throughout the central region 30. The apical taper 32 and coronal taper 34 are each between about 5°-15° relative to the central axis A. In bone applications these ranges represent critical boundaries, in that less than about 5° will result in too little radial compression, whereas greater than about 15° will result in too much radial compression. It is not necessary that the apical 32 and 34 coronal tapers be substantially equal, i.e., matched to one another. However, in the illustrated examples the apical 32 and 34 coronal tapers are each about 10° which has been found to produce optimal results. The central taper 36 is between about 0°-5° relative to the central axis A. In bone applications this range represents critical boundaries; less than about 0° will create a negative taper condition, whereas greater than about 5° will result in too large a variation in radial compression along the length of the central region 30 and/or too little radial compression adjacent the apical region 26. In the illustrated examples the central taper 36 is about 1° which has been found to produce optimal results. For non-dental orthopedic applications, somewhat larger taper ranges may be desired. For non-medical applications, still larger taper ranges may be considered.

In practice, the longitudinal lengths of the apical region 26, coronal region 28 and central region 30 can vary relative to the entire longitudinal length of the body. For example, in the illustrated embodiment of Figure 7, if the longitudinal length of the coronal region 28 is designated as one unit, the central region 30 is about two units in length and the apical region 26 is about 1⅓ units. By these rough measures, the entire longitudinal length of the body is thus about 4⅓ units. Naturally, the relative lengths of the apical region 26, coronal region 28 and central region 30 are highly adaptable to suit the intended application. That is to say, these dimensional relationships can be altered to suit the application and/or to achieve specific performance attributes. For example, the apical region 26 could be lengthened or shortened in relative terms; the coronal region 28 could be lengthened or shortened in relative terms, and the central region 30 could be lengthened or shortened in relative terms. Of course, many still further alternatives will become apparent to the skilled person in these arts. Notwithstanding the variable relative lengths, the outer form of the body should maintain a generally conical taper that enlarges toward the coronal end 24. A conical geometry is believed to support superior primary stability and loading protocols.

An internal tool socket 38 is disposed in the body, opening directly from the coronal end 24 as seen in Figure 5. A full profile of the tool socket 38 can be seen in Figure 7, extending through the coronal region 30 and well into the central region 30. These examples show the tool socket 38 in the form of a hex-point receptacle for coupling with a complimentary-shaped driver head (not shown). Of course, the form of the tool socket 38 will be matched to the application and the standards of the relevant industry/field of use. The tool socket 38 is formed as a pass-through feature enabling access to as recessed threaded section 40. The threaded section 40 in this example extends past the central region 30 and encroaches into the apical region 26. The threaded section 40 is adapted to connect with an abutment or other feature (not shown) to be subsequently attached to the implanted anchor 20.

As shown throughout the several views, at least one thread form protrudes from the body. The phrase "at least one" is used to underscore that in some contemplated embodiments the anchor 20 may be configured with double-lead or even triple-lead thread forms. However, in the illustrated examples the thread form is comprised of a single lead that winds helically there along in continuous turns from the apical region 26, through the entirety of the central region 30, and well into the coronal region 28. In most contemplated applications, the thread form will wind about the body in the right-hand direction according to most common conventions. Of course, if a particular application were to dictate a preference for left-hand windings, a complete mirror image of the anchor 20 would be expected to perform with similar functionality.

In Figure 4, the pitch of the thread form is indicated by construction line 42. The pitch 42 can be any suitable low angle, but in any case is generally constant along the length of the body. The illustrated examples depict a pitch 42 in the neighborhood of 5°, but of course that is merely one example.

As best seen in the cross-section of Figure 7, the thread form has a leading flank 44 disposed toward the apical region 26. That is, the leading flank 44 is the helical surface of the thread form that faces toward the apical end 22. The leading flank 44 maintains a generally constant leading flank angle LF that is indicated in Figure 7 by the construction line 46. The leading flank angle LF is measured from the central axis A on the apical end 22 side as being obtuse to facilitate insertion. The leading flank angle LF is preferably between about 110°-130° as measured toward the apical region 26. In the illustrated examples, the leading flank angle LF is shown at about 120° which has been found to provide suitable results.

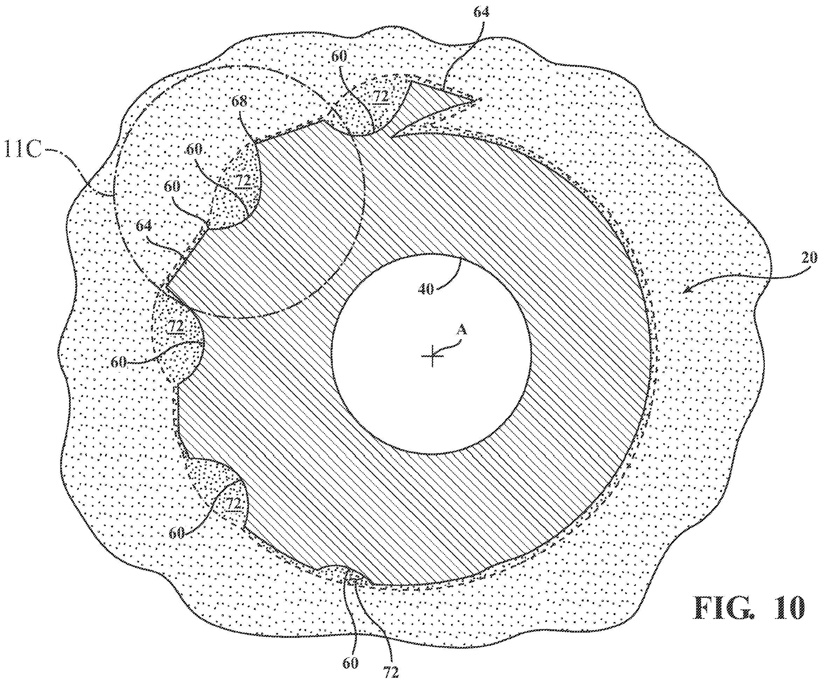

The thread form has a trailing flank 48 disposed toward the coronal region 28. The trailing flank 48 is the helical surface of the thread form that faces toward the coronal end 24. The trailing flank 48 maintains a generally constant trailing flank angle TF that is indicated in Figure 7 by the construction line 50. The trailing flank angle TF is measured from the central axis A on the coronal end 24 side as being acute in order to better resist pull-out and to avoid radial strain on the host bone under tensile loading. The trailing flank 48 is preferably between about 75°-85° as measured toward the coronal region 28. In the illustrated examples, the trailing flank angle TF is shown at about 80° which has been found to provide optimal results. Additional benefits attributable to the acute back-angle of the trailing flank angle TF will be described in connection with Figures 10-12.

The thread form has a crest 52, as is common with most thread forms. In this case, the crest 52 is truncated as perhaps best understood by considering the construction lines 46 and 50 in Figure 7. If the leading 44 and trailing 48 flanks were extended fully, they would meet in a sharp edge creatine a blade-like crest. However, in the illustrated examples the crest 52 has a width that is generally axial or longitudinal in disposition. As will be described subsequently, the crest 52 is specially configured to provide active functionality and novel attributes to the anchor 20.

According to standard screw thread nomenclature, the thread form can be seen having a minor diameter that is established by the body, and a major diameter established by the crest 52. The minor diameter corresponds to the root diameter of the thread form. The portion of the body between adjacent turns of the thread form comprises a root. The root has an axial root length which, in concert with the constant pitch, is generally equivalent along the length of the body. That is, at any point along the thread form, the axial root length will be generally the same. The thread form has a thread thickness. The thread thickness is the axial dimension between leading 44 and trailing 48 flanks as measured at the body. Figures 4 and 7 show that the thread thickness may be greater in the central region 30 than in either of the apical 26 and coronal 28 regions. Notably, even though thread thickness may decrease in the apical 26 and coronal 28 regions, the axial root length remains generally constant. Additional benefits attributable to the generally constant axial root length will be described in connection with Figures 10-12.

The terminal ends of the thread form are noteworthy. An apical transitional chamfer 54 is disposed between the blunt apical tip 22 and the start or leading end of the thread form. Similarly, a coronal transitional chamfer 56 is disposed between the flat coronal end 24 and the thread form. The apical transitional chamfer 54 helps to avoid snags and catches during installation. As can be seen in Figure 1, the coronal transitional chamfer 56 facilitates the in-growth of bone (or another host material) and helps to mechanically lock the anchor 20 *in situ.* The minor diameter and the major diameter of the thread form is substantially equal at the respective points of intersection with the apical 54 and coronal 56 transition chamfers. The point of intersection at the coronal transitional chamfer 56 is visible in Figure 4. Figure 7 probably offers the best depiction of the point of intersection at the apical transitional chamfer 54. In these, it can be seen that the radial projection of the thread form in each of the apical 26 and coronal 28 regions diminishes steadily. At each transitional chamfer 54, 56, the thread form terminates and also blends smoothly into the root or body of the anchor 20, thus causing the minor and major diameters of the thread form to merge or coalesce. Especially at the coronal end 24, the diminishing radial projection of the thread form is very beneficial in bone implant applications where it is important not to over-stress the outer layer of hard cortical bone (see Figure 1).

Figures 3, 4 and 6 clearly shown that the apical region 26 may include at least one self-tapping notch 58. In the illustrated examples, a plurality (three, in this case) of equally spaced self-tapping notches 58 are employed. Each self-tapping notch 58 axially bisects a plurality of the turns of the thread form and penetrates into the body, creating a pocket for bone chips (or other host material chips) during the screw-in process. Each notch 58 has a generally planar cutting face that carves away the host material to make a path for the thread form as the anchor 20 is advanced by turning into the prepared hole.

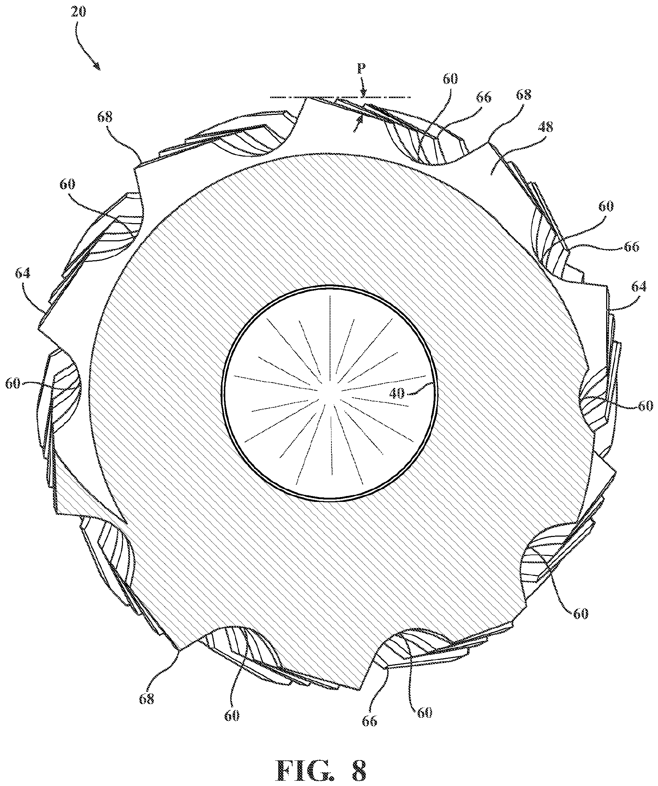

The central region 30 of the anchor 20 includes an array of flutes 60. Any number of flutes 60 is possible. The cross-sectional view of Figure 8 shows that ten flutes 60 are formed on the anchor 20 of the illustrated examples. The flutes 60 are preferably equally spaced from one another in the circumferential direction. In the example where an anchor 20 includes ten flutes (Figure 8), each flute 60 will be circumferentially offset from the next adjacent flute by 36° (0.2π radians). Regardless of the number of flutes 60, each extends longitudinally along the length of the central region 30, as perhaps best seen in Figure 3. That is, the flutes 60 are primarily confined to the central region 30; except for perhaps minimal encroachments the flutes 60 do not extend into either of the apical 26 or coronal 28 regions.

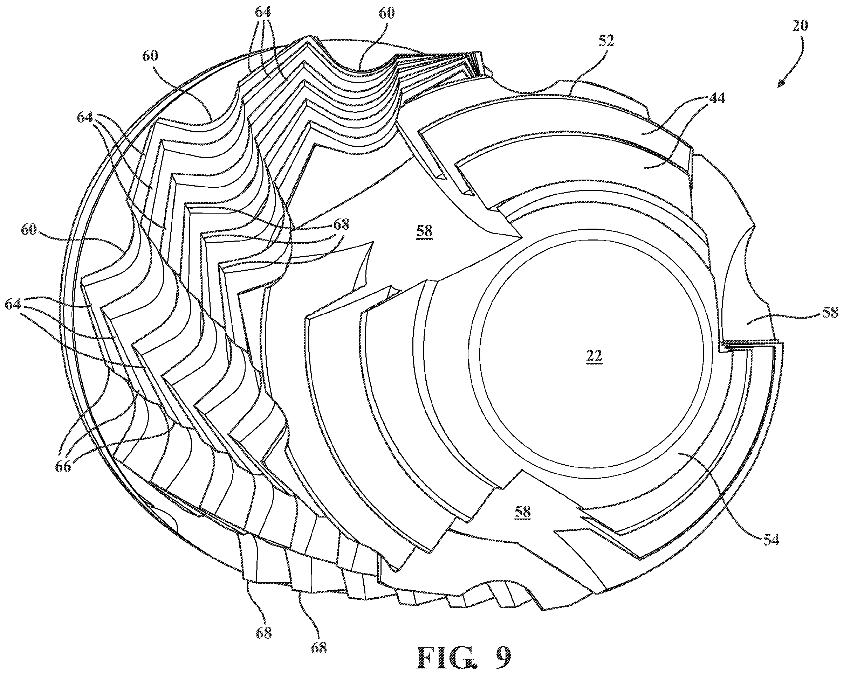

The flutes 60 are not continuous uninterrupted valleys. Instead, each flute 60 is composed of a plurality of distinct and isolated flute segments separated from one another by the gap between windings in the thread form. That is to say, each flute 60 is actually formed by an alignment of several stand-alone flute segments perhaps like a walking path can be formed by a series of individual stepping-stones. The aforementioned helical root intersects the flutes 60 with each revolution, thereby dividing each flute 60 into a plurality of aligned segments which is easily appreciated from the perspective of Figure 9. Thus, a collection of flute segments establishes a single flute 60. Each isolated flute segment is formed as a scallop in the crest 52 of the thread form. If it is assumed for convenience that each flute 60 is composed of an average of seven flute segments, and if the anchor 20 has ten flutes 60, then the total number of flutes segments will be about seventy uniformly distributed about the exterior of the central region 30 of the anchor 20.

Each isolated flute segment within a common flute 60 is circumferentially offset from the next adjacent isolated flute segment so as to form a helical twist. The helical twist is indicated in Figure 4 by construction line 62. If each flute segment is circumferentially offset from the next adjacent isolated flute segment by -10°, for example, the helical twist of each flute 60 will be -10°. The negative value of the helical twist (e.g., -10°) denotes a left-hand direction. That is, the flute 60 can be seen to run or propagate in a direction opposite to the rotation direction needed to screw the anchor 20 into its prepared hole.

One advantage of a left-hand twist in the flutes 60 is appreciated in the context of anchor 20 removal. Most host materials, and especially in cases where the host material is live bone, host materials will migrate into the flutes 60 after placement. This migration could be motivated by recovery (spring-back) in elastic and semi-elastic materials due to stresses introduced into the host material, and possibly also ingrowth in the case of living host materials like bone and trees. Host material that enters the flutes 60 will mechanically lock the anchor 20 in place. Any attempt to unscrew the anchor 20 will be fought by the host material inside the flute 60 which, because of the left-hand twist, will only urge the anchor 20 more deeply.

In the illustrated examples, each flute 60 has a generally constant flute depth and generally consistent flute width/span. That is to say, the size and shape of each flute segment is generally the same. However, this is not a requirement. In some contemplated embodiments the flutes 60 could be formed with varying depths and/or widths, and/or with variable helical twist 62.

The central region 30 also includes an array of condensing ramps 64. Each condensing ramp 64 is disposed along the crest 52 of the thread form between two circumferentially adjacent flute segments. That is, the condensing ramps 64 are located on the crest 52 between adjacent neighboring flutes 60 within the central region 30. Each condensing ramp 64 has a low leading edge 66 and a high trialing edge 68 arranged in the right-hand direction. That is to say, the adjectives "leading" and "trailing" are based on rotation of the anchor 20 in a right-hand direction as when being screwed into a prepared hole. The low leading edge 66 will precede the high tailing edge 68 in rotation like a ramp or wedge. As best seen in Figure 8, the condensing ramp 64 is pitched at an angle P between about 10°-30° relative to a tangent line. In the illustrated examples the pitch angle P is about 20° which has been found to produce optimal results.

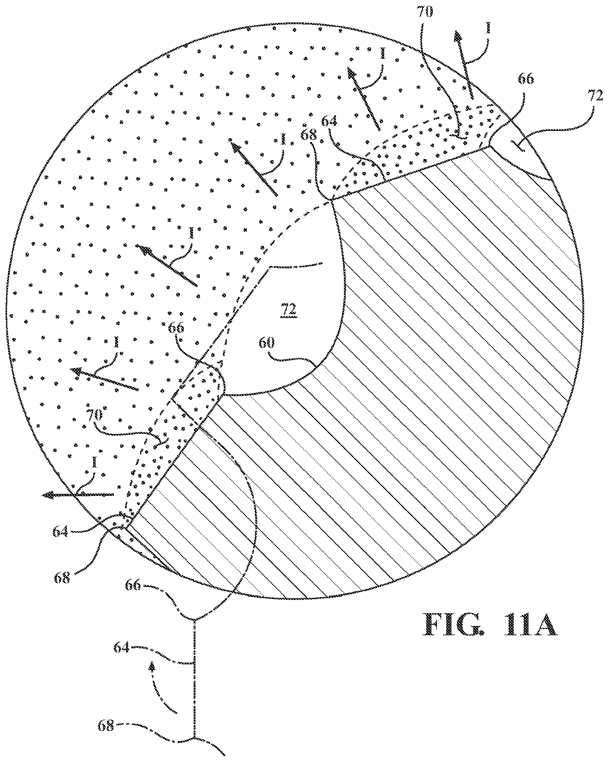

Each condensing ramp 64 is configured to apply a localized compressive strain to the interior surface of the host material with a densifying action while the anchor 20 is screwed into the prepared hole. More specifically, the helical portion of the host material that coincides with the thread form will be directly affected by the condensing ramps 64; gaps between the thread form will not be directly affected. Figure 11A graphically depicts two condensing ramps 64 straddling a flute segment which, starting from the phantom position, are rotated clockwise to the final resting position shown in solid lines. As the condensing ramps 64 drag across the interior surface of the hole, they produce localized areas of highly agitated, highly compressed bone (or other host) material, which will be referred to hereafter as super-activated zones 70. The condensing ramps 64 also induce stresses I in the surrounding host material. The induced stresses I are represented by directional arrows to indicate the direction in which the bone (or other host material) is displaced by the wiping action of the condensing ramps 64. Figure 11A is thus intended to depict, in highly simplified fashion, the moment at which the anchor 20 comes to rest upon reaching full depth at the time of placement. A small void 72 is expected where each flute segment comes to rest.

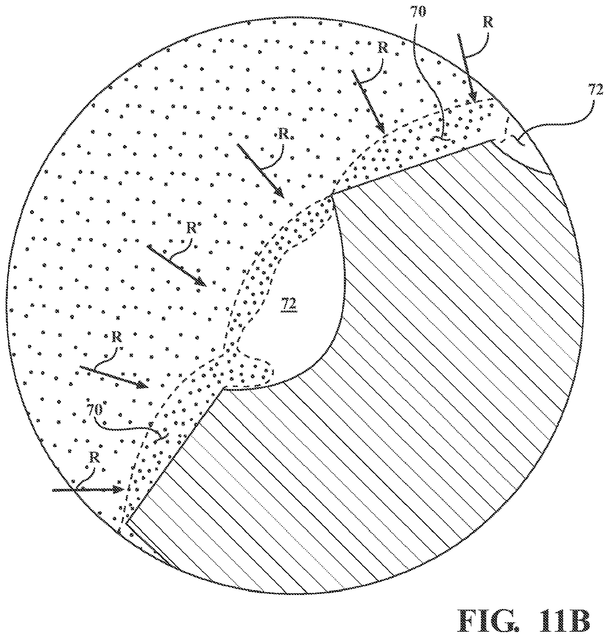

Figure 11B is similar to Figure 11A but is a portrayal after the passage of time - perhaps moments after Figure 11A or perhaps days or even weeks. Two motivating factors influence the host material to begin filling the voids 72 of the flutes 60, which enable the voids to be considered healing chambers 72. The healing chambers 72 serve as incubator sites to rapidly achieve secondary stability. One motivating factor is the reaction R of the host material to the previously induced stresses I (Figure 11A). Reaction forces R are represented by directional arrows to indicate the direction in which the bone (or another host material) elastically shifts after the anchor 20 stops rotating. Induced stresses I that are within the host material's ability to deform elastically will reverse themselves as reaction forces R and promptly return toward the un-deformed condition once the stress I is removed. The reaction forces R will thus cause the surrounding bone or host material to constrict about the anchor 20 and fill the voids 72 of the flutes 60. The absence of host material in the voids 72 means there is little-to-no resistance thus welcoming the movement of reaction bone into the healing chambers 72. That is to say, the healing chambers 72 will effectively draw the bone (or another host material). It has been said that nature abhors a vacuum. By analogy, the healing chambers 72 will naturally encourage and promote the inflow of bone to fill the voids.

The other motivating factor is the due to the super-activated zones 70. In living host materials, e.g., bone, when the induced stresses I exceed the bone's ability to deform elastically, the bone will deform and change shape permanently by plastic deformation. In bone, the permanent change in shape may be associated with micro-cracks that allow energy release, a compromise that is a natural defense against complete fracture. This also produces blood flow, which leads to clots forming in the voids of the flutes 60. All of this agitation activates the bone's natural healing properties leading to rapid growth of new bone into the flutes 60. Thus, the super-activated zones 70 promote the natural healing properties in the human body to accelerate recovery and improve osseointegration, especially into the healing chambers 72 formed where each flute segment comes to rest once the anchor 20 is fully seated.

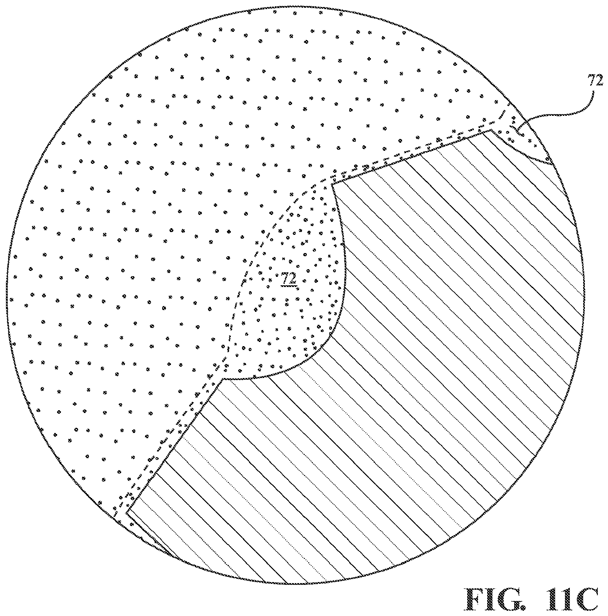

Figure 11C is similar to Figure 11B but is a portrayal after the passage of considerable time - perhaps many weeks after Figure 11B or perhaps months later. Figure 11C is taken from Figure 10 which depicts the entire anchor 20 in cross-section. Surrounding the anchor 20 is a strong, dense layer of new bone growth, spurred on by the super-activated zones 70 and reaction forces R. The healing chambers 72 are now filled with solid new bone that lock the anchor 20 in place. Earlier, it was estimated that the illustrated anchor 20 may have approximately seventy flute segments in total. That means seventy discrete healing chambers 72, evenly distributed about the entire external central region 30, were concurrent new bone growth is incubated to accelerate the osseointegration process. This wide and even distribution of isolated healing chambers 72 super-accelerates the healing process.

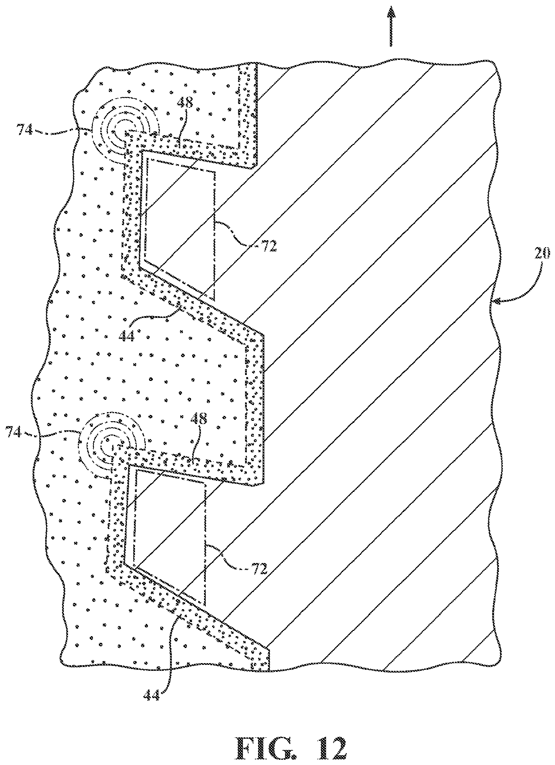

Figure 12 is a fragment of a longitudinal cross-section showing two turns of the thread form in an implanted anchor 20. This view is helpful to visualize that the host material trapped in the gap between turns of the thread form remains generally undisturbed as the anchor 20 is screwed into position. Specifically, the generally constant pitch 42 of the thread form combined with the generally constant axial root length means that the section of host material between turns of the thread form will not have been directly interrupted by installing the anchor 20. In contrast, the helical band of host material engaged by the crest 52 of the thread form is highly agitated and disturbed by screwing the anchor 20 into place due to the condensing ramps 64. However, this highly agitated and disturbed band of host material does not directly affect the host material located in the gap between turns of the thread form. As a result, the natural structural integrity of the host material remains largely intact in the gap between turns of the thread form.

Figure 12 is thus helpful to understand that when an anchor 20 is initially placed (e.g., Figures 11A and 11B), the host material trapped in the gap between turns of the thread form serves to provide a favorably high level of initial, or primary, stability to the anchor 20. During these moments and days following placement of the anchor 20, the healing chambers 72 have not had adequate time to fill and achieve osseointegration. It is during this stage that the host material in the gap between turns of the thread form primarily secures the anchor 20 in position. The previously mentioned acute back-angle of the trailing flank angle TF can be seen to provide benefits particularly during these earliest stages following anchor 20 placement.

Figure 12 indicates a pull-out (tensile) force urging the anchor 20 from its host. Under these circumstances, the acute angle of the trailing flank 48 imposes a concentration of stress 74 in the host material as far away from the body (root) of the anchor 20 as possible. Stress concentrations are represented by the concentric array of broken circles 74 in Figure 12. Furthermore, this location of stress concentration 74 will be more likely to coincide (spatially) with any new bone growth that may have been able to form from the super-activated zones 70, thus enabling the greatest possible resistance to pull-out. If one imagines the trailing flank angle TF were obtuse, such as found in a common Acme style thread form, the stress concentration 74 under tensile (pull-out) loading would occur directly adjacent the body (root) of the anchor 20. The anchor 20 would, in that imagined configuration, be less suited to resist pull-out forces.

However, once sufficient time is allotted for new bone growth via the super-activated zones 70 and healing chambers 72, the new bone growth provides substantial enhanced secondary stability for the anchor 20 that enables full loading under all normal conditions. Even after full osseointegration, the acute angle of the trailing flank 48 functions like a helical barb that contributes to secondary stability.

Returning to Figure 7, a crosshatched area is shown on the left side of the anchor 20 to indicate a controlled compression zone 76. The controlled compression zone 76 is defined between the body of the anchor 20 (i.e., the root of the thread form) and the crest 52 of the thread form, from apical 22 to coronal 24 end. The design of this invention reveals careful attention to manage the effects this zone 76 on the host material. It can be seen that in the apical region 26, the controlled compression zone 76 gently and progressively displaces the host material, both at the root and crest 52 of the thread form. This is represented by the differential between the minor diameter and the major diameter of the thread form. As stated previously, these diameters are substantially equal at the intersection with the apical transition chamfer 54. However, as the radial projection of the thread form increases relative to the root, the relative displacement of host material changes in the helical band affected by the crest 52 of the thread form. In other words, although both the root and crest 52 of the thread form are pushing the host material outwardly in the apical region 26 of the controlled compression zone 76, the action of the crest 52 has a greater magnitude.

In the central region 30 of the controlled compression zone 76, both the root and crest 52 of the thread form are pushing the host material outwardly at about the same rate as dictated by the central taper 36 (Figure 4). Thus, the apical region 26 initiates an aggressive/rapid expansion in the controlled compression zone 76, but throughout the central region 30 the continued displacement of host material is relatively mild. Entering the coronal region 28 of the controlled compression zone 76, a compression inversion takes places wherein the expansion attributable to the root rapidly increased by the expansion attributable to the crest 52 decreases until the relative displacement is zero at the coronal transition chamfer 56. One purpose of this configuration is to equalize the radial stresses in the host material at the coronal transition chamfer 56. In human bone, for example, a hard layer of cortical bone typically lays on the surface with soft cancellous bone on the interior. See Figure 1. By equalizing the radial stresses in the area of the hard cortical bone, stress fractures are less likely to develop around the implanted anchor 20.

The foregoing invention has been described in accordance with the relevant legal standards, thus the description is exemplary rather than limiting in nature. Variations and modifications to the disclosed embodiment may become apparent to those skilled in the art and fall within the scope of the invention. Features of interest, in addition to the appended claims, include:

An anchor wherein the apical taper is substantially equal to the coronal taper. A thread form may wind in the right-hand direction, said thread form having a pitch, said pitch being generally constant along the length of said body. Furthermore, said thread form may have a minor diameter established by said body and a major diameter established by said crest, the portion of said body between adjacent turns of said thread form comprising a root of said thread form, said root having an axial root length, said root length being generally equivalent along the length of said thread form, said thread form having a thread thickness, said thread thickness being greater in said central region than in said apical and coronal regions. The minor diameter and said major diameter of said thread form may be substantially equal at the termination of said thread form adjacent said coronal end.

A particular form comprises an anchor of the type screwed into a prepared hole in live bone, said anchor comprising: a body extending longitudinally along a central axis, said body having an apical region and a coronal region, a central region of said body extending between said apical region and said coronal region, said body having an apical taper throughout said apical region, said body having a coronal taper throughout said coronel region, said body having a central taper throughout said central region, said central taper being about 1° relative to said central axis, said apical taper being between about 10° relative to said central axis, said apical taper substantially equal to said coronal taper; an internal tool socket disposed in said body adjacent said coronal region; at least one thread form protruding from said body and winding helically there along in continuous turns from said apical region to said coronal region, said thread form winding in the right-hand direction, said thread form having a pitch, said pitch being generally constant along the length of said body, said thread form having a leading flank disposed toward said apical region, said leading flank having a leading flank angle of about 120° measured toward said apical region, said thread form having a trailing flank disposed toward said coronal region, said trailing flank having a trailing flank angle about 80° measured toward said coronal region, said thread form having a truncated crest, said thread form having a minor diameter established by said body and a major diameter established by said crest, the portion of said body between adjacent turns of said thread form comprising a root, said root having an axial root length, said root length being generally equivalent along the length of said thread form, said thread form having a thread thickness, said thread thickness being greater in said central region than in said apical and coronal regions; said apical region having at least one self-tapping notch therein axially bisecting a plurality of the turns of said thread form; said central region including an array of flutes, each said flute extending longitudinally along the length of said central region, each said flute being composed of a plurality of distinct and isolated flute segments, said isolated flute segments formed in said crest of said thread form, each said isolated flute segment within said flute being circumferentially offset from the next adjacent isolated flute segment to form a helical twist, said helical twist having a left-hand direction, each said flute having a generally constant flute depth; said central region including an array of condensing ramps, each said condensing ramp disposed along said crest of said thread form between two circumferentially-adjacent flute segments, each said condensing ramp having a low leading edge and a high trialing edge in the right-hand direction, said condensing ramp being pitched about 20° relative to a tangent line, each said condensing ramp configured to apply a localized compressive strain to the interior surface of the host bone with a densifying action while said anchor is screwed into the prepared hole; and said coronal region having a flat end, a coronal transitional chamfer disposed between said flat end and said thread form, said minor diameter and said major diameter of said thread form being substantially equal at the intersection thereof with said coronal transition chamfer.

## Claims

1. An anchor (20) of the type screwed into a prepared hole in a host material, said anchor (20) comprising:
a body extending longitudinally along a central axis, said body having an apical region (26) and a coronal region (28), a central region (30) of said body extending between said apical region (26) and said coronal region (28),
at least one thread form protruding from said body and winding helically there along in continuous turns from said apical region (26) to said coronal region (28), said thread form having a crest, said central region (30) including an array of flutes (60), each said flute (60) extending longitudinally along the length of said central region (30),
each said flute (60) being composed of a plurality of distinct and isolated flute segments formed in said crest of said thread form,
wherein within said central region (30) said thread form has a leading flank (44) disposed toward said apical region (26) and a trailing flank (48) disposed toward said coronal region (28), said leading flank (44) having an obtuse leading flank angle measured toward said apical region (26), said trailing flank (48) having an acute trailing flank angle measured toward said coronal region (28).

2. The anchor of Claim 1, wherein each said isolated flute segment within said flute is circumferentially offset from the next adjacent isolated flute segment to form a helical twist in said flute.

3. The anchor of Claim 2, wherein said helical twist in each said flute has a left-hand direction.

4. The anchor of Claim 2, wherein each said flute has a generally constant flute depth.

5. The anchor of Claim 1, wherein within said central region said thread form includes an array of condensing ramps, each said condensing ramp disposed along said crest of said thread form between two circumferentially-adjacent flute segments, each said condensing ramp configured to apply a localized compressive strain to the host material with a densifying action while said anchor is screwed into the prepared hole.

6. The anchor of Claim 5, wherein each said condensing ramp has a low leading edge and a high trialing edge in the right-hand direction, said condensing ramp being pitched between about 10°-30° relative to a tangent line.

7. The anchor of Claim 1, wherein said leading flank angle is between about 110°-130° measured toward said apical region, said trailing flank angle is between about 75°-85° measured toward said coronal region.

8. The anchor of Claim 1, wherein said body has an apical taper throughout said apical region, said body having a coronal taper throughout said coronal region, said body having a central taper throughout said central region, said central taper being between about 0°-5° (1°) relative to said central axis, said apical taper being between about 5°-15° (10°) relative to said central axis, said apical taper substantially equal to said coronal taper.

9. The anchor of Claim 1, wherein said thread form winds in the right-hand direction, said thread form having a pitch, said pitch being generally constant along the length of said body.

10. The anchor of Claim 1, wherein said thread form has a minor diameter established by said body and a major diameter established by said crest, the portion of said body between adjacent turns of said thread form comprising a root of said thread form, said root having an axial root length, said root length being generally equivalent along the length of said thread form, said thread form having a thread thickness, said thread thickness being greater in said central region than in said apical and coronal regions.

11. The anchor of Claim 10, wherein said minor diameter and said major diameter of said thread form are substantially equal at the termination of said thread form adjacent said coronal end.

12. A bone anchor, of the type screwed into a prepared hole in host bone, comprising the anchor of claim 1, further comprising:
said body having an apical taper throughout said apical region, said apical taper being between about 5°-15° relative to said central axis, said body having a coronal taper throughout said coronal region, said coronal taper being between about 5°-15° relative to said central axis, said body having a central taper throughout said central region, said central taper being between about 0°-5° relative to said central axis,
said crest being a truncated crest and said thread form having a leading flank disposed toward said apical region and a trailing flank disposed toward said coronal region, said leading flank having a leading flank angle of about between about 110°-130° measured toward said apical region, said trailing flank having a trailing flank angle between about 75°-85° measured toward said coronal region.

13. The anchor of Claim 12, wherein each said isolated flute segment within said flute is circumferentially offset from the next adjacent isolated flute segment to form a left-hand helical twist.

14. The anchor of Claim 12, wherein within said central region said thread form includes an array of condensing ramps, each said condensing ramp disposed along said crest of said thread form between two circumferentially-adjacent flute segments, each said condensing ramp configured to apply a localized compressive strain to the host bone with a densifying action while said anchor is screwed into the prepared hole.

15. The anchor of Claim 14, wherein each said condensing ramp has a low leading edge and a high trialing edge in the right-hand direction, said condensing ramp being pitched between about 10°-30° relative to a tangent line.

## Patentansprüche

1. Ein Anker (20) des in eine vorbereitete Bohrung in einem Wirtsmaterial eingeschraubten Typs, der Anker (20) umfassend:
Einen Körper, der sich in Längsrichtung entlang einer Mittelachse erstreckt, wobei der Körper einen apikalen Bereich (26) und einen koronalen Bereich (28) aufweist, ein mittlerer Bereich (30) des Körpers erstreckt sich zwischen dem apikalen Bereich (28) und dem koronalen Bereich (28),
mindestens eine Gewindeform, die aus dem Körper herausragt und dort spiralförmig in kontinuierlichen Windungen von dem apikalen Bereich (26) zu dem koronalen Bereich (28) verläuft,
die Gewindeform hat einen Kranz, der mittlere Bereich (30) beinhaltet eine Anordnung von Rillen (60), die sich in Längsrichtung entlang der Länge des mittleren Bereichs (30) erstrecken,
jede Rille (60) besteht aus einer Vielzahl ausgeprägter und isolierter Rillensegmente, die in dem Kranz der Gewindeform gebildet sind,
wobei innerhalb des mittleren Bereichs (30) die Gewindeform eine vordere Flanke (44) hat, die in Richtung des apikalen Bereichs (26) angeordnet ist, und eine hintere Flanke (48), die in Richtung des koronalen Bereichs (28) angeordnet ist, die vordere Flanke (44) hat einen stumpfen Winkel an der vorderen Flanke, der in Richtung des apikalen Bereichs (26) gemessen wird, die hintere Flanke (48) hat einen spitzen Winkel an der hinteren Flanke, der in Richtung des koronalen Bereichs (28) gemessen wird.

2. Der Anker nach Anspruch 1, wobei jedes isolierte Rillensegment innerhalb der Rille umlaufend von dem nächsten benachbarten isolierten Rillensegment versetzt ist, um eine spiralförmige Windung in der Rille zu bilden.

3. Der Anker nach Anspruch 2, wobei die spiralförmige Windung in jeder Rille eine Linksrichtung aufweist.

4. Der Anker nach Anspruch 2, wobei jede Rille eine im Allgemeinen konstante Rillentiefe aufweist.

5. Der Anker nach Anspruch 1, wobei die Gewindeform innerhalb des mittleren Bereichs eine Anordnung schräger Komprimierungsflächen aufweist, jede der schrägen Komprimierungsflächen ist entlang des Kranzes der Gewindeform zwischen zwei umlaufenden, benachbarten Rillensegmenten angeordnet, jede schräge Komprimierungsfläche ist konfiguriert, eine lokale Formänderung durch den Druck auf das Wirtsmaterial mit einer verdichtenden Wirkung anzuwenden, während der Anker in die vorbereitete Bohrung eingeschraubt wird.

6. Der Anker nach Anspruch 5, wobei jede der schrägen Komprimierungsflächen eine niedrige Vorderkante und eine hohe Hinterkante in Rechtsrichtung aufweist und die schräge Komprimierungsfläche zwischen ungefähr 10°-30° im Verhältnis zu einer Tangente geneigt ist.

7. Der Anker nach Anspruch 1, wobei der Winkel der vorderen Flanke zwischen ungefähr 110°-130°, gemessen in Richtung des apikalen Bereichs, liegt und der Winkel der hinteren Flanke zwischen ungefähr 75°-85°, gemessen in Richtung des koronalen Bereichs, gemessen wird.

8. Der Anker nach Anspruch 1, wobei der Körper eine apikale Verjüngung über den koronalen apikalen Bereich hinweg aufweist und der Körper eine koronale Verjüngung über den gesamten Bereich aufweist, wobei der Körper eine mittlere Verjüngung über den mittleren Bereich aufweist, die mittlere Verjüngung zwischen ungefähr 0°-5° (1°) im Verhältnis zu der Mittelachse beträgt, die apikale Verjüngung zwischen ungefähr 5°-15° (10°) im Verhältnis zu der Mittelachse beträgt und die apikale Verjüngung im Wesentlichen gleich ist wie die koronale Verjüngung.

9. Der Anker nach Anspruch 1, wobei die Gewindeform in Rechtsrichtung dreht, die Gewindeform eine Neigung aufweist und die Neigung im Allgemeinen konstant entlang der Länge des Körpers verläuft.

10. Der Anker nach Anspruch 1, wobei die Gewindeform einen geringeren Durchmesser aufweist, der durch den Körper bestimmt wird, und einen größeren Durchmesser, der durch den Kranz bestimmt wird, der Abschnitt des Körpers zwischen den benachbarten Drehungen der Gewindeform umfasst eine Wurzel der Gewindeform, die Wurzel hat eine axiale Wurzellänge, die Wurzellänge ist im Allgemeinen entlang der Länge der Gewindeform gleich, die Gewindeform hat eine Gewindestärke, die Gewindestärke ist in dem mittleren Bereich größer als in dem apikalen und koronalen Bereich.

11. Der Anker nach Anspruch 10, wobei der kleinere Durchmesser und der größere Durchmesser der Gewindeform am Ende der Gewindeform am koronalen Ende im Wesentlichen gleich sind.

12. Ein Knochenanker des in eine vorbereitete Bohrung in einem Wirtsknochen eingeschraubten Typs, umfassend den Anker nach Anspruch 1 und ferner umfassend:
den Körper mit einer apikalen Verjüngung über den apikalen Bereich, die apikale Verjüngung beträgt zwischen ungefähr 5°-15° im Verhältnis zu der Mittelachse, der Körper weist eine koronale Verjüngung über den koronalen Bereich auf, die koronale Verjüngung beträgt zwischen ungefähr 5°-15° im Verhältnis zur Mittelachse,
der Körper hat eine mittlere Verjüngung über den mittleren Bereich, die mittlere Verjüngung beträgt zwischen ungefähr 0°-5° im Verhältnis zur Mittelachse,
der Kranz ist ein kegelstumpfartiger Kranz und die Gewindeform weist eine vordere Flanke auf, die in Richtung des apikalen Bereichs angeordnet ist, und eine hintere Flanke, die in Richtung des koronalen Bereichs angeordnet ist, die hintere Flanke hat einen hinteren Flanken-Winkel zwischen ungefähr 110°-130°, gemessen in Richtung des apikalen Bereichs, die hintere Flanke hat einen hinteren Flanken-Winkel zwischen ungefähr 75°-85°, gemessen in Richtung des koronalen Bereichs.

13. Der Anker nach Anspruch 12, wobei jedes isolierte Rillensegment innerhalb der Rille umlaufend von dem nächsten benachbarten isolierten Rillensegment versetzt ist, um eine linksdrehende spiralförmige Windung in der Rille zu bilden.

14. Der Anker nach Anspruch 12, wobei die Gewindeform innerhalb des mittleren Bereichs eine Anordnung schräger Komprimierungsflächen aufweist, jede der schrägen Komprimierungsflächen ist entlang des Kranzes der Gewindeform zwischen zwei umlaufenden, benachbarten Rillensegmenten angeordnet, jede schräge Komprimierungsfläche ist konfiguriert, eine lokale Formänderung durch den Druck auf das Wirtsmaterial mit einer verdichtenden Wirkung anzuwenden, während der Anker in die vorbereitete Bohrung eingeschraubt wird.

15. Der Anker nach Anspruch 14, wobei jede der schrägen Komprimierungsflächen eine niedrige Vorderkante und eine hohe Hinterkante in Rechtsrichtung aufweist und die schräge Komprimierungsfläche zwischen ungefähr 10°-30° im Verhältnis zu einer Tangente geneigt ist.

## Revendications

1. Ancrage (20) du type vissé dans un trou préparé dans un matériau hôte, ledit ancrage (20) comprenant :
un corps s'étendant longitudinalement le long d'un axe central, ledit corps présentant une région apicale (26) et une région coronale (28), une région centrale (30) dudit corps s'étendant entre ladite région apicale (28) et ladite région coronale (28),
au moins une forme de filetage faisant saillie à partir dudit corps et s'enroulant de manière hélicoïdale en tours continues depuis ladite région apicale (26) jusqu'à ladite région coronale (28),
ladite forme de filetage présentant une crête, ladite région centrale (30) comportant un réseau de cannelures (60) s'étendant longitudinalement sur la longueur de ladite région centrale (30),
chacune desdites cannelures (60) étant composée d'une pluralité de segments de cannelure distincts et isolés formés dans ladite crête de ladite forme de filetage,
dans lequel, à l'intérieur de ladite région centrale (30), ladite forme de filetage présente un flanc avant (44) disposé vers ladite région apicale (26) et un flanc arrière (48) disposé vers ladite région coronale (28), ledit flanc avant (44) présentant un angle de flanc avant obtus mesuré vers ladite région apicale (26), le flanc arrière (48) présentant un angle de flanc arrière aigu mesuré vers ladite région coronale (28).

2. Ancrage selon la revendication 1, dans lequel chacun desdits segments de cannelure isolés à l'intérieur de ladite cannelure est décalé suivant la circonférence par rapport au segment de cannelure isolé adjacent suivant pour former une torsade hélicoïdale dans ladite cannelure.

3. Ancrage selon la revendication 2, dans lequel ladite torsade hélicoïdale dans chacune desdites cannelures présente une direction vers la gauche.

4. Ancrage selon la revendication 2, dans lequel chacune desdites cannelures présente une profondeur de cannelure généralement constante.

5. Ancrage selon la revendication 1, dans lequel, à l'intérieur de ladite région centrale, ladite forme de filetage comporte un réseau de rampes de condensation, chacune desdites rampes de condensation étant disposée le long de ladite crête de ladite forme de filetage entre deux segments de cannelure adjacents suivant la circonférence, chacune desdites rampes de condensation est conçue pour appliquer une contrainte de compression localisée au matériau hôte avec une action de densification pendant que ledit ancrage est vissé dans le trou préparé.

6. Ancrage selon la revendication 5, dans lequel chacune desdites rampes de condensation présente un bord avant bas et un bord arrière haut dans la direction de droite, ladite rampe de condensation étant inclinée entre environ 10° et 30° par rapport à une tangente.

7. Ancrage selon la revendication 1, dans lequel ledit angle de flanc avant est compris entre environ 110° et 130° mesuré vers ladite région apicale, ledit angle de flanc arrière est compris entre environ 75° et 85° mesuré vers ladite région coronale.

8. Ancrage selon la revendication 1, dans lequel ledit corps présente un cône apical sur l'étendue de ladite région apicale coronale, ledit corps présentant un cône coronal sur l'étendue de ladite région, ledit corps présentant un cône central sur l'étendue de ladite région centrale, ledit cône central étant compris entre environ 0° et 5° (1°) par rapport audit axe central, ledit cône apical étant compris entre environ 5° et 15° (10°) par rapport audit axe central, ledit cône apical étant sensiblement égal audit cône coronal.

9. Ancrage selon la revendication 1, dans lequel ladite forme de filetage s'enroule dans la direction droite, ladite forme de filetage présentant un pas, ledit pas étant généralement constant sur la longueur dudit corps.

10. Ancrage selon la revendication 1, dans lequel ladite forme de filetage présente un diamètre mineur établi par ledit corps et un diamètre majeur établi par ladite crête, la partie dudit corps entre des tours adjacents de ladite forme de filetage comprenant une racine de ladite forme de filetage, ladite racine présentant une longueur de racine axiale, ladite longueur de racine étant généralement équivalente sur la longueur de ladite forme de filetage, ladite forme de filetage présentant une épaisseur de filetage, ladite épaisseur de filetage étant plus grande dans ladite région centrale que dans lesdites régions apicale et coronale.

11. Ancrage selon la revendication 10, dans lequel ledit petit diamètre et ledit grand diamètre de ladite forme de filetage sont sensiblement égaux à la terminaison de ladite forme de filetage adjacente à ladite extrémité frontale

12. Ancrage osseux, du type vissé dans un trou préparé dans un os hôte, comprenant l'ancrage selon la revendication 1, comprenant en outre :
ledit corps présentant un cône apical sur l'étendue de ladite région apicale, ledit cône apical étant compris entre environ 5° et 15° par rapport audit axe central, ledit corps présentant un cône coronal sur l'étendue de ladite région coronale, ledit cône coronal étant compris entre environ 5° et 15° par rapport audit axe central,
ledit corps présentant un cône central sur l'étendue de ladite région centrale, ledit cône central étant compris entre environ 0° et 5° par rapport audit axe central,
ladite crête étant une crête tronquée et ladite forme de filetage présentant un flanc avant disposé vers ladite région apicale et un flanc arrière disposé vers ladite région frontale, ledit flanc avant présentant un angle de flanc avant compris entre environ 110° et 130° mesuré vers ladite région apicale, ledit flanc arrière présentant un angle de flanc arrière compris entre environ 75° et 85° mesuré vers ladite région coronale.

13. Ancrage selon la revendication 12, dans lequel chacun desdits segments de cannelure isolés à l'intérieur de ladite cannelure est décalé suivant la circonférence par rapport au segment de cannelure isolé adjacent suivant pour former une torsade hélicoïdale vers la gauche.

14. Ancrage selon la revendication 12, dans lequel, à l'intérieur de ladite région centrale, ladite forme de filetage comporte un réseau de rampes de condensation, chacune desdites rampes de condensation étant disposée le long de ladite crête de ladite forme de filetage entre deux segments de cannelure adjacents suivant la circonférence, chacune desdites rampes de condensation est conçue pour appliquer une contrainte de compression localisée à l'os hôte avec une action de densification pendant que ledit ancrage est vissé dans le trou préparé.

15. Ancrage selon la revendication 14, dans lequel chacune desdites rampes de condensation présente un bord avant bas et un bord arrière haut dans la direction de droite, ladite rampe de condensation étant inclinée entre environ 10° et 30° par rapport à une tangente.
